# EUROPEAN PATENT SPECIFICATION

(11) **EP 1 539 731 B1**
(45) Date of publication and mention of the grant of the patent: **02.01.2008**
(21) Application number: 03738863.4
(22) Date of filing: 15.07.2003
(51) Int. Cl.: C07D 333/34, A61K 31/381, A61P 29/00

(54) **NOVEL COMPOUNDS**
NEUE VERBINDUNGEN
NOUVEAUX COMPOSES

(30) Priority: 19.07.2002 SE 0202279
(43) Date of publication of application: 15.06.2005
(73) Proprietor: AstraZeneca AB, 151 85 Södertälje (SE)
(72) Inventor: METE, Antonio, Loughborough, Leicestershire LE11 5RH (GB); WALTERS, Iain, Loughborough, Leicestershire LE11 5RH (GB)
(86) International application number: PCT/SE2003/001215
(87) International publication number: WO 2004/009580

(56) References cited:
- EP-A1- 0 373 836
- WO-A1-01/62704
- WO-A1-01/62713

## Description

### Field of the Invention

The present invention relates to novel heteroarylalkylamine derivatives, processes for their preparation, compositions containing them and their use in therapy.

### Background of the Invention

Nitric oxide is produced in mammalian cells from L-arginine by the action of specific nitric oxide synthases (NOSs). These enzymes fall into two distinct classes - constitutive NOS (cNOS) and inducible NOS (iNOS). At the present time, two constitutive NOSs and one inducible NOS have been identified. Of the constitutive NOSs, an endothelial enzyme (eNOS) is involved with smooth muscle relaxation and the regulation of blood pressure and blood flow, whereas the neuronal enzyme (nNOS) appears to be involved in the regulation of various biological functions. Inducible NOS has been particularly implicated in the pathogenesis of inflammatory diseases. Regulation of these enzymes should therefore offer considerable potential in the treatment of a wide variety of disease states (J. E. Macdonald, Ann. Rep. Med. Chem., 1996, 31, 221 - 230).

Considerable effort has been expended in efforts to identify compounds that act as specific inhibitors of one or more isoforms of the enzyme nitric oxide synthase. The use of such compounds in therapy has also been widely claimed.

Phenylheteroalkylamine derivatives which are inhibitors of nitric oxide synthase are described in WO 01/62713 A1 and WO 01/62704 A1. Propanamines having activity as serotonin and norepinephrine uptake inhibitory activity have been described in EP 373836 a1.

### Disclosure of the invention

According to the present invention, there is provided a compound of formula (I) 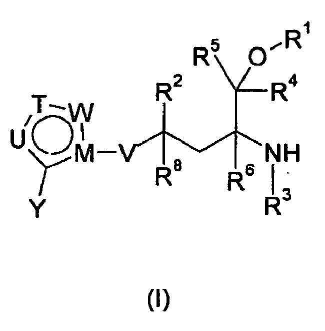 wherein:
Y represents C1 to 4 alkyl, C1 to 4 alkoxy, halogen, CN, C=CH, NO₂, CH₂OH, CHO, COCH₃, NH₂, NHCHO, NHCOCH3 or NHSO₂CH₃; said alkyl or alkoxy group being optionally further substituted by one or more fluorine atoms;
T, U and W independently represent CX, N, NR¹³, O or S(O)ₘ, except that at least one of T, U and W must represent a heteroatom and except that not more than one of T, U and W may represent NR¹³, O or S(O)ₘ; m represents an integer 0, 1 or 2; and each X group independently represents H, C1 to 4 alkyl, C1 to 4 alkoxy, halogen, OH, SH, CN, C≡CH, N(R ¹⁴)₂, NO₂, CH₂OH, CHO, COCH₃ or NHCHO; said alkyl or alkoxy group being optionally further substituted by one or more fluorine atoms;
V represents O or S(O)ₙ;
n represents an integer 0, 1 or 2;
M represents C;
R¹ and R⁸ independently represent H or Me.
R² represents C1 to 4 alkyl, C2 to 4 alkenyl, C2 to 4 alkynyl, C3 to 6 cycloalkyl or a 4 to 8 membered saturated heterocyclic ring incorporating one heteroatom selected from O, S and N; any of said groups being optionally further substituted by C 1 to 4 alkyl, C 1 to 4 alkoxy, C 1 to 4 alkylthio, C3 to 6 cycloalkyl, habgen or phenyl; said phenyl group being optionally further substituted by one or more substituents selected independently from halogen, C1 to 4 alkyl, C1 to 4 alkoxy, CF₃, OCF₃, CN or NO₂;
or R² represents phenyl or a five or six membered aromatic heterocyclic ring containing 1 to 3 heteroatoms independently selected from O, S and N; said phenyl or aromatic heterocyclic ring being optionally substituted by one or more substituents selected independently from halogen, C1 to 4 alkyl, C1 to 4 alkoxy, OH, CN, NO₂ or NR⁹R¹⁰; said alkyl or alkoxy group being optionally further substituted by one or more fluorine atoms;
R³ represents H, C 1 to 4 alkyl or C3 to 6 cycloalkyl; said alkyl group being optionally substituted by C1 to 4 alkoxy, halogen, hydroxy, NR¹¹R¹², phenyl or a five or six membered aromatic or saturated heterocyclic ring containing 1 to 3 heteroatoms independently selected from O, S and N; said phenyl or aromatic heterocyclic ring being optionally further substituted by halogen, C1 to 4 alkyl, C1 to 4 alkoxy, CF₃, OCF₃, CN or NO₂;
R⁷ and R¹⁴ independently represent H or C1 to 2 alkyl;
R⁴, R⁵, R⁶, R⁹, R¹⁰, R¹¹ and R¹² independently represent H or C1 to 4 alkyl;
R¹³ represents H, C 1 to 4 alkyl, CHO, COCH₃, SO₂CH₃ or CF₃;
or a pharmaceutically acceptable salt thereof.

The compounds of formula (I) may exist in enantiomeric forms. It is to be understood that all enantiomers, diastereomers, racemates and mixtures thereof are included within the scope of the invention.

It will be recognised that compounds of formula (I) wherein U, T or W represents CX and X represents OH may exist in the alternative keto tautomeric form. Similarly, compounds of formula (I) wherein U, T or W represents CX and X represents NH may exist in the alternative imino tautomeric form. And similarly, compounds of formula (I) wherein U, T or W represents CX and X represents SH may exist in the alternative thioketo tautomeric form. It is to be understood that all such possible tautomeric forms and mixtures thereof are included within the scope of the invention.

The compounds of formula (I) and their pharmaceutically acceptable salts have the advantage that they are inhibitors of the enzyme nitric oxide synthase (NOS). In particular, the compounds of formula (I) and their pharmaceutically acceptable salts have the advantage that they are inhibitors of the inducible isoform of the enzyme nitric oxide synthase (iNOS).

The invention further provides a process for the preparation of compounds of formula (I) or a pharmaceutically acceptable salt, enantiomer or racemate thereof.

According to the invention there is also provided a compound of formula (I), or a pharmaceutically acceptable salt thereof, for use as a medicament.

Another aspect of the invention provides the use of a compound of formula (I) or a pharmaceutically acceptable salt thereof, in the manufacture of a medicament, for the treatment or prophylaxis of diseases or conditions in which inhibition of nitric oxide synthase activity is beneficial.

Another aspect of the invention provides the use of a compound of formula (I) or a pharmaceutically acceptable salt thereof, in the manufacture of a medicament, for the treatment or prophylaxis of diseases or conditions in which inhibition of inducible nitric oxide synthase activity is beneficial.

A more particular aspect of the invention provides the use of a compound of formula (I) or a pharmaceutically acceptable salt thereof, in the manufacture of a medicament, for the treatment or prophylaxis of inflammatory disease.

Another aspect of the invention provides the use of a compound of formula (I) or a pharmaceutically acceptable salt thereof, in the manufacture of a medicament, for the treatment or prophylaxis of pain.

According to the invention, there is also provided a method of treating, or reducing the risk of, diseases or conditions in which inhibition of nitric oxide synthase activity is beneficial which comprises administering to a person suffering from or at risk of, said disease or condition, a therapeutically effective amount of a compound of formula (I) or a pharmaceutically acceptable salt thereof.

More particularly, there is also provided a method of treating, or reducing the risk of, inflammatory disease in a person suffering from or at risk of, said disease, wherein the method comprises administering to the person a therapeutically effective amount of a compound of formula (I) or a pharmaceutically acceptable salt thereof.

The compounds of the present invention may also be used advantageously in combination with a second pharmaceutically active substance; particularly in combination with a cyclooxygenase inhibitor; more particularly in combination with a selective inhibitor of the inducible isoform of cyclooxygenase (COX-2). Thus, in a further aspect of the invention there is provided the use of a compound of formula (I) or a pharmaceutically acceptable salt thereof, in combination with a COX-2 inhibitor for the treatment of inflammation, inflammatory disease and inflammatory related disorders. And there is also provided a method of treating, or reducing the risk of, inflammation, inflammatory disease and inflammatory related disorders in a person suffering from or at risk of, said disease or condition, wherein the method comprises administering to the person a therapeutically effective amount of a compound of formula (I) or a pharmaceutically acceptable salt thereof in combination with a COX-2 inhibitor.

In one embodiment, V in formula (I) represents S(O)ₙ and n represents 0.

In another embodiment, Y in formula (I) represents CN.

In one embodiment, R¹ in formula (I) represents H.

In another embodiment, R² in formula (I) represents optionally substituted phenyl or a five or six membered aromatic heterocyclic ring containing 1 to 3 heteroatoms independently selected from O, S and N. In a further embodiment, R² in formula (I) represents optionally substituted phenyl, pyridyl, thienyl, isoxazolyl, isothiazolyl or thiazolyl. In a yet further embodiment, R² in formula (I) represents optionally substituted phenyl.

In one embodiment, R³ in formula (I) represents H.

In another embodiment R⁴, R⁵ and R⁶ in formula (I) each represent H.

In a particular embodiment, the compounds of formula (I) have the absolute stereochemistry as shown:

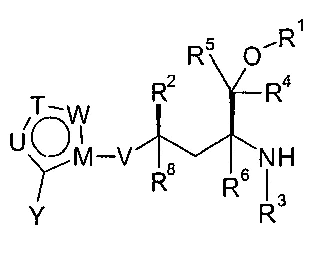

Particular compounds of the invention include:
3-[[(1*R*,3*S*)-3-amino-4-hydroxy-1-phenylbutyl]thio]-2-thiophenecarbonitrile;
3-[[(1*R*,3*S*)-3-amino-4-hydroxy-1-phenylbutyl]thio]-5-methyl-2-thiophenecarbonitrile;
and pharmaceutically acceptable salts thereof.

Unless otherwise indicated, the term "C1 to 4 alkyl" referred to herein denotes a straight or branched chain alkyl group having from 1 to 4 carbon atoms. Examples of such groups include methyl, ethyl, n-propyl, i-propyl, n-butyl, i-butyl and t-butyl.

Unless otherwise indicated, the term "C3 to 6 cycloalkyl" referred to herein denotes a cycloalkyl group having from 3 to 6 carbon atoms. Examples of such groups include cyclopropyl, cyclopentyl and cyclohexyl.

Unless otherwise indicated, the term "C2 to 4 alkenyl" referred to herein denotes a straight or branched chain alkyl group having from 2 to 4 carbon atoms incorporating at least one carbon-carbon double bond. Examples of such groups include ethenyl, propenyl and butenyl.

Unless otherwise indicated, the term "C2 to 4 alkynyl" referred to herein denotes a straight or branched chain alkyl group having from 2 to 4 carbon atoms incorporating at least one carbon-carbon triple bond. Examples of such groups include ethynyl, propynyl, and butynyl.

Unless otherwise indicated, the term "C1 to 4 alkoxy" referred to herein denotes a straight or branched chain alkoxy group having from 1 to 4 carbon atoms. Examples of such groups include methoxy, ethoxy, n-propoxy, i-propoxy and t-butoxy.

The term "C1 to 4 alkylthio" is to be interpreted analogously.

Unless otherwise indicated, the term "halogen" referred to herein denotes fluoro, chloro, bromo and iodo.

Examples of a 4 to 8 membered saturated heterocyclic ring incorporating one heteroatom selected from O, S or N include pyrrolidine, piperidine, tetrahydrofuran and perhydroazepine.

Examples of a five or six membered aromatic heterocyclic ring containing 1 to 3 heteroatoms independently selected from O, S and N include furan, thiophene, pyridine, thiazole, imidazole, oxazole, triazole, oxadiazole, thiadiazole and pyrimidine.

Examples of a five or six membered saturated heterocyclic ring containing 1 to 3 heteroatoms independently selected from O, S and N include pyrrolidine, tetrahydrofuran, piperidine and piperazine.

Examples of a "C1 to 4 alkyl or C1 to 4 alkoxy optionally further substituted by one or more fluorine atoms" include CH₂F, CHF₂, CF₃, CF₃CF₂, CF₃CH₂, CH₂FCH₂, CH₃CF₂, CF₃CH₂CH₂, OCF₃ and OCH₂CF₃.

According to the invention, we further provide a process for the preparation of compounds of formula (I), or a pharmaceutically acceptable salt, enantiomer or racemate thereof which comprises:
(a) reaction of a compound of formula (II) 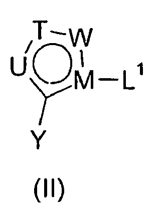 wherein T, U, W, Y and M are as defined in formula (I) and L¹ represents a leaving group, with a compound of formula (III) 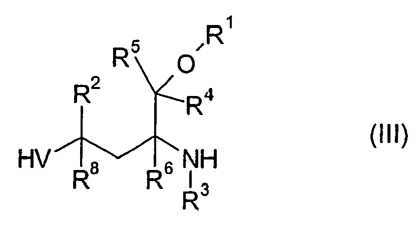 wherein R¹, R², R³, R⁴, R⁵, R⁶, R⁸ and V are as defined in formula (I); or
(b) reaction of a compound of formula (IV) 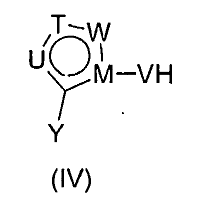
wherein T, U, W, M, Y and V are as defined in formula (I), with a compound of formula (V) 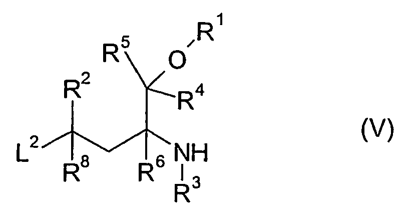 wherein R¹, R², R³, R⁴, R⁵, R⁶ and R⁸ are as defined in formula (I) and L² is a leaving group;
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

In process (a), the reaction is performed by treating a nucleophile of formula (III) with an electrophile of formula (II) in an inert solvent. Suitable leaving groups L¹ include sulphonates and halides, particularly fluoride or chloride. The reaction is generally performed in the presence of a non-nucleophilic base such as sodium hydride or caesium carbonate. Suitable organic solvents are those such as N,N-dimethylformamide, N-methyl-2-pyrrolidinone, tetrahydrofuran, acetonitrile and dimethylsulfoxide. The reaction is generally conducted at a temperature between 0 °C and the boiling point of the solvent.

In process (b), the reactants (IV) and (V) are coupled together in a suitable inert solvent such as tetrahydrofuran using, for example, Mitsunobu conditions. Thus, for example, the reactants are treated with a phosphine derivative and an azo derivative at a suitable temperature, generally between 0 °C and the boiling point of the solvent. Suitable phosphine derivatives include triphenylphosphine and tributylphosphine. Suitable azo derivatives include diethyl azodicarboxylate, diisopropyl azodicarboxylate and 1,1'-(azodicarbonyl)dipiperidine. Suitable leaving groups L² include hydroxy.

Alternatively in process (b), the reaction is performed by treating a nucleophile of formula (IV) with an electrophile of formula (V) in an inert solvent. Suitable leaving groups L² include sulphonates and halides, particularly chloride or bromide. The reaction is generally performed in the presence of a non-nucleophilic base such as sodium hydride or caesium carbonate. Suitable organic solvents are those such as N,N-dimethylformamide, N-methyl-2-pyrrolidinone, tetrahydrofuran and dimethylsulfoxide. The reaction is generally conducted at a temperature between 0 °C and the boiling point of the solvent.

It will be apparent to a person skilled in the art that in the above processes it may be desirable or necessary to protect an amine or hydroxyl or other potentially reactive group.

Suitable protecting groups and details of processes for adding and removing such groups may be found by reference to the standard text "Protective Groups in Organic Synthesis", 3rd Edition (1999) by Greene and Wuts.

In one preferred embodiment, amine groups are protected as carbamate derivatives, for example, as t-butyloxycarbamates.

In another particularly preferred embodiment, the amine and hydroxyl groups of compounds wherein R¹ represents hydrogen are protected simultaneously as a cyclic carbamate, such as in formula (VI), or as a cyclic hemi-aminal as in formula (VII).

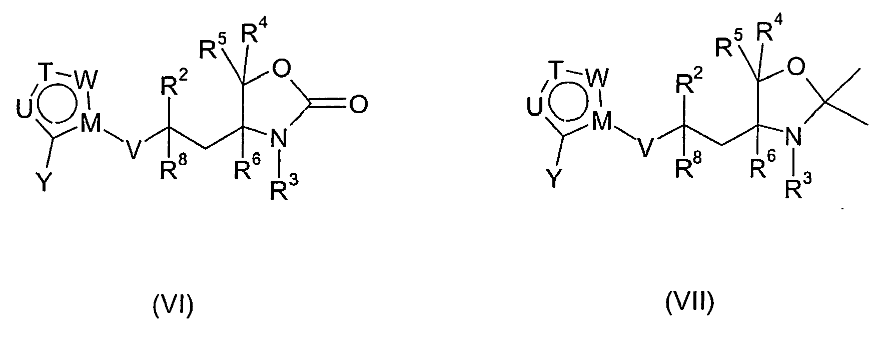

Specific examples of the use of protecting groups are given in the Examples section.

The present invention includes compounds of formula (I) in the form of salts, in particular acid addition salts. Suitable salts include those formed with both organic and inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, phosphoric, citric, tartaric, lactic, pyruvic, acetic, succinic, fumaric, maleic, methanesulphonic and benzenesulphonic acids.

Salts of compounds of formula (I) may be formed by reacting the free base, or a salt, enantiomer or racemate thereof, with one or more equivalents of the appropriate acid. The reaction may be carried out in a solvent or medium in which the salt is insoluble or in a solvent in which the salt is soluble, for example, water, dioxane, ethanol, tetrahydrofuran or diethyl ether, or a mixture of solvents, which may be removed *in vacuo* or by freeze drying. The reaction may also be a metathetical process or it may be carried out on an ion exchange resin.

Compounds of formula (III) wherein R⁸ represents H may be prepared by reaction of a compound of formula (VIII) 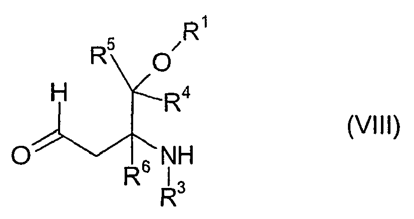 wherein R¹, R³, R⁴, R⁵ and R⁶ are as defined in formula (I),
with an organometallic derivative, R² - M, wherein R² is as defined in formula (I) and M represents a metallic residue such as lithium or magnesium-halide. The resulting compound of formula (III) wherein V represents oxygen may then be subsequently converted into compounds of formula (III) wherein V represents sulphur.

Alternatively, compounds of formula (III) may be prepared by reaction of an amide of formula (IX) 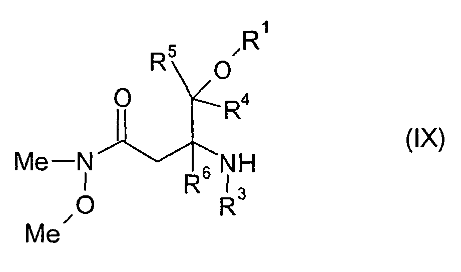 wherein R¹, R³, R⁴, R⁵ and R⁶ are as defined in formula (I),
with an organometallic derivative, R² - M, wherein R² is as defined in formula (I) and M represents a metallic residue such as lithium or magnesium-halide, followed by reduction of the resulting ketone to the corresponding alcohol (III).

Compounds of formulae (II), (IV), (VIII) and (LX) are either known or may be prepared by conventional methods that will be readily apparent to the man skilled in the art.

Intermediate compounds may be used as such or in protected form. Protecting groups and details of processes for their removal may be found by reference to the standard text "Protective Groups in Organic Synthesis", 3rd Edition (1999) by Greene and Wuts.

The compounds of the invention and intermediates thereto may be isolated from their reaction mixtures and, if necessary further purified, by using standard techniques.

The compounds of formula I may exist in enantiomeric forms. Therefore, all enantiomers, diastereomers, racemates and mixtures thereof are included within the scope of the invention. The various optical isomers may be isolated by separation of a racemic mixture of the compounds using conventional techniques, for example, fractional crystallisation, or HPLC.

Intermediate compounds may also exist in enantiomeric forms and may be used as purified enantiomers, diastereomers, racemates or mixtures.

The compounds of formula (I), and their pharmaceutically acceptable salts, enantiomers and racemates, are useful because they possess pharmacological activity in animals. In particular, the compounds are active as inhibitors of the enzyme nitric oxide synthase. More particularly, they are inhibitors of the inducible isoform of the enzyme nitric oxide synthase and as such are predicted to be useful in therapy, for example, as anti- inflammatory agents. They may also have utility as inhibitors of the neuronal isoform of the enzyme nitric oxide synthase. In general, compounds of formula (I) and their pharmaceutically acceptable salts have the advantage that they show good selectivity for the inhibition of iNOS and/or nNOS in comparison to the inhibition of the endothelial isoform, eNOS.

The compounds and their pharmaceutically acceptable salts are indicated for use in the treatment or prophylaxis of diseases or conditions in which synthesis or oversynthesis of nitric oxide synthase forms a contributory part. In particular, the compounds are indicated for use in the treatment of inflammatory conditions in mammals including man.

Conditions that may be specifically mentioned are:
osteoarthritis, rheumatoid arthritis, rheumatoid spondylitis, gouty arthritis and other arthritic conditions, inflamed joints;
eczema, psoriasis, dermatitis or other inflammatory skin conditions such as sunburn; inflammatory eye conditions including uveitis, glaucoma and conjunctivitis;
lung disorders in which inflammation is involved, for example, asthma, bronchitis, chronic obstructive pulmonary disease, pigeon fancier's disease, farmer's lung, acute respiratory distress syndrome;
bacteraemia, endotoxaemia (septic shock), aphthous ulcers, gingivitis, pyresis, pain, meningitis and pancreatitis;
conditions of the gastrointestinal tract including inflammatory bowel disease, Crohn's disease, atrophic gastritis, gastritis varialoforme, ulcerative colitis, coeliac disease, regional ileitis, peptic ulceration, irritable bowel syndrome, reflux oesophagitis, damage to the gastrointestinal tract resulting from infections by, for example, *Helicobacter pylori,* or from treatments with non-steroidal anti-inflammatory drugs;
and other conditions associated with inflammation.

The compounds may also be useful in the treatment of cancer.

The compounds will also be useful in the treatment and alleviation of acute pain or persistent inflammatory pain or neuropathic pain or pain of a central origin.

We are particularly interested in the conditions inflammatory bowel disease, rheumatoid arthritis, osteoarthritis, chronic obstructive pulmonary disease, pain and cancer.

The compounds of formula (I) and their pharmaceutically acceptable salts may also be useful in the treatment or prophylaxis of diseases or conditions in addition to those mentioned above. For example, the compounds may be useful in the treatment of atherosclerosis, cystic fibrosis, hypotension associated with septic and/or toxic shock, in the treatment of dysfunction of the immune system, as an adjuvant to short-term immunosuppression in organ transplant therapy, in the control of onset of diabetes, in the maintenance of pancreatic function in diabetes, in the treatment of vascular complications associated with diabetes and in co-therapy with cytokines, for example TNF or interleukins.

The compounds of formula (I) may also be useful in the treatment of hypoxia, for example in cases of cardiac arrest and stroke, neurodegenerative disorders including nerve degeneration and/or nerve necrosis in disorders such as ischaemia, hypoxia, hypoglycaemia, epilepsy, and in external wounds (such as spinal cord and head injury), hyperbaric oxygen convulsions and toxicity, dementia, for example pre-senile dementia, Alzheimer's disease and AIDS-related dementia, Sydenham's chorea, Parkinson's disease, Tourette's syndrome, Huntington's disease, amyotrophic lateral sclerosis, multiple sclerosis, muscular dystrophy, Korsakoffs disease, imbecility relating to a cerebral vessel disorder, sleeping disorders, schizophrenia, depression, pain, autism, seasonal affective disorder, jet-lag, depression or other symptoms associated with premenstrual syndrome (PMS), anxiety and septic shock. Compounds of formula (I) may also be expected to show activity in the prevention and reversal of drug addiction or tolerance such as tolerance to opiates and diazepines, treatment of drug addiction, treatment of migraine and other vascular headaches, neurogenic inflammation, in the treatment of gastrointestinal motility disorders and in the induction of labour.

We are particularly interested in the conditions stroke, Alzheimer's disease, Parkinson's disease, multiple sclerosis, schizophrenia, migraine, septic shock and pain.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

For the above mentioned therapeutic indications, the dosage administered will, of course, vary with the compound employed, the mode of administration and the treatment desired. However, in general, satisfactory results are obtained when the compounds are administered at a dosage of the solid form of between 1 mg and 2000 mg per day.

The compounds of formula (I), and pharmaceutically acceptable derivatives thereof, may be used on their own, or in the form of appropriate pharmaceutical compositions in which the compound or derivative is in admixture with a pharmaceutically acceptable adjuvant, diluent or carrier. Administration may be by, but is not limited to, enteral (including oral, sublingual or rectal), intranasal, inhalation, intravenous, topical or other parenteral routes. Conventional procedures for the selection and preparation of suitable pharmaceutical formulations are described in, for example, "Pharmaceuticals - The Science of Dosage Form Designs", M. E. Aulton, Churchill Livingstone, 1988. The pharmaceutical composition preferably comprises less than 80% and more preferably less than 50% of a compound of formula (I), or a pharmaceutically acceptable salt thereof.

According to the invention, we further provide a pharmaceutical composition comprising a compound of formula (I), or a pharmaceutically acceptable salt thereof, in admixture with a pharmaceutically acceptable adjuvant, diluent or carrier.

There is also provided a process for the preparation of such a pharmaceutical composition which comprises mixing the ingredients.

The compounds of formula (I), and pharmaceutically acceptable derivatives thereof, may also be advantageously used in combination with one of the following therapies: NSAIDS, COX-2 inhibitors, Paracetamol, Tramadol, Corticosteroids, Glucosamine, Doxycyclin, Pralnacasan, MMP inhibitors or Coll-3 inhibitors. The compound of formula (I) and the combination therapy may either be formulated together within the same pharmaceutical composition for administration in a single dosage unit, or each component may be individually formulated such that separate dosages may be administered either simultaneously or sequentially.

The invention is illustrated, but in no way limited, by the following examples:

The following abbreviations are used:- DMSO (dimethylsulfoxide), DMF *(N,N-*dimethylformamide), THF (tetrahydrofuran).

### Example 1

### 3-[[(1R,3S)-3-Amino-4-hydroxy-1-phenylbutyl]thio]-2-thiophenecarbonitrile oxalate

### a) Phenylmethyl (3S)-3-[[(1,1-dimethylethoxy)carbonyllaminol-4-hydroxy-butanoate

A solution of 4-(phenylmethyl) *N*-[(1,1-dimethylethoxy)carbonyl]-1-(2,5-dioxo-1-pyrrolidinyl)) L-aspartate (75.0 g) in THF (200 ml) was added over 1 h to a suspension of sodium borohydride (6.84 g) in THF (60 ml) and water (90 ml) at -5 °C (internal temperature kept below 15 °C). Further sodium borohydride (6.8 g in two batches) was added and the mixture was stirred for 45 minutes. The mixture was poured into cold, stirred, half-saturated ammonium chloride solution (600 ml) and extracted twice with ethyl acetate. The organic layers were dried (MgSO₄) and evaporated to give the sub-title compound as a waxy solid (56.24 g).

MS APCI +ve ^{m}/z 210 [M+H-BOC]⁺.
¹H NMR 300MHz (CDCl₃) 7.41-7.27 (5H, m), 5.24-5.10 (3H, m), 4.15-3.96 (1H, m), 3.71 (2H, d), 2.69 (2H, d), 1.44 (9H, s).

### b) Phenylmethyl (4S)-3-[(1,1-dimethylethoxy)carbonyl]-2,2-dimethyl-4-oxazolidine-acetate

2-Methoxypropene (46 ml) was added over 20 minutes to a solution of the product from step (a) (74.88 g), 2,2-dimethoxypropane (30 ml) and p-toluenesulphonic acid (1.21 g) in dichloromethane (300 ml) at 0 °C and stirred at 0 °C for 1 h and at 20 °C for 1 h. 1M sodium hydrogen carbonate solution was added and the mixture was extracted with dichloromethane (3 x 200 ml). The organic layers were dried (MgSO₄) and evaporated to give a colourless oil which was dissolved in toluene (300 ml), 2,2-dimethoxypropane (45 ml) and p-toluenesulphonic acid (1.2 g) added and the mixture was heated at 80 °C for 2 h. On cooling, potassium carbonate was added and the mixture was extracted twice with ethyl acetate. The organic layers were dried (MgSO₄) and evaporated to give the sub-title compound (83.8 g) as a pale yellow oil.

¹H NMR 300MHz (CDCl₃) 7.36-7.28 (5H, m), 5.12 (2H, d), 4.38-3.97 (2H, m), 3.84 (1H, d), 3.05-2.48 (2H, m), 1.62-1.50 (6H, m), 1.46 (9H, s).

### c) (4S)-3-[(1,1-Dimethylethoxy)carbonyl}-2,2-dimethyl-4-oxazolidineacetic acid

A suspension of 10% palladium on carbon (3.8 g) and the product from step (b) (83.8 g) in ethanol (250 ml) was stirred under hydrogen (4 atmospheres pressure) for 3.5 h (5.3 1 hydrogen uptake). The mixture was filtered through celite and evaporated. Ethyl acetate (100 ml) and 1M potassium carbonate solution (200 ml) were added and the organic layer was separated and further extracted with 1M potassium carbonate solution (40 ml) and 1M sodium hydrogen carbonate solution (40 ml). The aqueous layers were washed with ethyl acetate, combined and acidified at 0 °C by dropwise addition of 4M hydrochloric acid (130 ml). The aqueous was extracted with ethyl acetate (3 x 200 ml) and the organic layers were dried (MgSO₄) and evaporated to give the sub-title compound as a pale orange gum (56.24 g), which slowly crystallised.

¹H NMR 300MHz (CDCl₃) 4.33-4.12 (1H, m), 4.09-4.00 (1H, m), 3.86 (1H, d), 3.02-2.77 (1H, m), 2.62-2.50 (1H, m), 1.62-1.54 (6H, m), 1.53 (9H, s).

### d) 1-Dimethylethyl (4S)-4-[2-(methoxymethylamino)-2-oxoethyl]-2,2-dimethyl-3-oxazolidinecarboxylate

*N,*O-Dimethylhydroxylamine hydrochloride (21.4 g), EDCI (41.94 g), N-methylmorpholine (24 ml) and DMAP (26.4 g) were added to a solution of the product from step (c) (59.2 g) in dichloromethane (400 ml) at 0 °C and then stirred at 20 °C for 18 h. 2M hydrochloric acid (200 ml) was added, the organic layer was separated and the aqueous was further extracted twice. The organic layers were washed with 2M hydrochloric acid (50 ml) and sodium hydrogen carbonate solution (2 x 100 ml), combined, dried (MgSO₄) and evaporated to give the sub-title compound (60.2 g).

MS APCI +ve ^{m}/z 303 [M+H]⁺.
¹H NMR 300MHz (CDCl₃) 4.38-4.19 (1H, m), 4.08 (1H, dd), 3.87 (1H, t), 3.70 (3H, s), 3.17 (3H, s), 3.07-2.45 (2H, m), 1.63-1.42 (15H, m).

### e) 1,1-Dimethylethyl (4S) 2,2-dimethyl-4-(2-oxo-2-phenylethyl)-3-oxazolidinecarboxylate

Phenyl magnesium bromide (231 ml, 1M in THF) was added over 15 minutes to a solution of the product from step (d) (60.1 g) in THF (360 ml) at -10 to-5 °C and then stirred for 2 h. Further phenyl magnesium bromide (7 ml, 3M in ether) was added and stirred at 0 °C for 1 h then quenched by the addition of saturated ammonium chloride solution (250 ml) and 2M hydrochloric acid (150 ml). The mixture was extracted three times with ethyl acetate and the organic layers were washed with brine, combined, dried (MgSO₄) and evaporated to give the sub-title compound (64.8 g) as an off-white solid.

¹H NMR 300MHz (CDCl₃) 7.98 (2H, d), 7.64-7.40 (3H, m), 4.50-4.35 (1H, m), 4.15-4.05 (1H, m), 3.88-3.65 (2H, m), 3.49-3.36 and 3.25-3.01 (1H, m), 1.70-1.35 (15H, m).

### f) 1,1-Dimethylethyl (4S)-4-[(2S)-2-hydroxy-2-phenylethyl]-2,2-dimethyl 3-oxazolidinecarboxylate

Borane (176 ml, 1M in THF) was added to a solution of (R) methyl-CBS-oxazaborolidine (16 ml, 1M in toluene) in THF (20 ml) and cooled to -20 °C. A solution of the product from step (e) (64.6 g) in THF (200 ml) was added over 1.5 h, keeping the internal temperature below -15 °C, and then stirring at this temperature for 22 h. Methanol (40 ml) was added slowly and the solution was evaporated and then azeotroped with methanol to give a pale yellow oil (69 g). Ether and 1M potassium hydrogen sulphate solution (20 ml) were added and the mixture was filtered and evaporated. Purification by chromatography (silica, 40-60 petrol/ether as eluent) gave the sub-title compound (37.4 g) as a white solid.

¹H NMR 400MHz (CDCl₃) 7.4-7.2 (5H, m), 4.88 (1H, d), 4.65 (1H, m), 4.35 (1H, m), 4.0 (1H, m), 3.65 (1H, d), 2.1-2 (1H, m), 1.85-1.95 (1H, m), 1.6 (3H, s), 1.49 (12H, s).

Further elution gave the (4*S*, 2R) isomer as a white solid (20.0 g).

¹H NMR 400MHz (CDCl₃) 7.4-7.3 (5H, brs), 4.77-4.73 (1H, m), 4.3-3.7 (3H, m), 2.2-2 (2H, m), 1.6-1.4 (15H, m).

### g) 1,1-Dimethylethyl (4S) 4-[(2R)-2-(benzoylthio)-2-phenylethyl]-2,2-dimethyl-3-oxazolidinecarboxylate

Diisopropyl azodicarboxylate (21.5 ml) in THF (20 ml) was added dropwise to a solution of triphenylphosphine (28.73 g) in THF (230 ml) at -10°C and the white suspension was stirred for 30 minutes. A solution of the major product (4*S*, 2*S*) from step (f) (17.58 g) and thiobenzoic acid (12.8 ml) in THF (100 ml) was added over 45 minutes at -10°C and then stirred at -10 °C to 4 °C for 18 h. The solvent was removed *in vacuo,* ether added and stirred until a precipitate formed. The mixture was filtered and the solids washed with isohexane/ether (1:1). The solution was washed with aqueous sodium hydrogen carbonate solution and the aqueous layer extracted with ether. The combined organic layers were dried (MgSO₄), evaporated and purified by chromatography (silica, 40-60 petrol/dichloromethane (1:1 then 0:1) as eluent) to give a solid. This was crystallised from isohexane at -78°C to give the sub-title compound (14.76 g) as a white solid.

¹H NMR 300MHz (CDCl₃) 7.93 (2H, d), 7.61-7.21 (8H, m), 4.79 (1H, dt), 4.18-3.83 (3H, m), 2.64-2.35 (1H, m), 2.23-2.09 (1H, m), 1.62-1.41 (15H, m).

### h) 1,1-Dimethylethyl (4S)-4-[(2R)-2-mercapto-2-phenylethyl]-2,2-dimethyl-3-oxazolidinecarboxylate

The product from step (g) (20.0 g) was dissolved in 7M ammonia in methanol (500 ml) and stirred at room temperature under nitrogen for 6 h. The solution was evaporated to dryness and the residue purified by chromatography (silica, 20% diethyl ether/isohexane) to give the sub-title compound (16.0g) as a white solid.

MS APCI +ve ^{m}/_{z} 238 (M+H-BOC)⁺.

### j) 1,1-Dimethylethyl (4S)-4-[(2R)-2-[(2-cyano-3-thienyl)thio]-2-phenylethyl]-2,2-dimethyl-3-oxazolidinecarboxylate

The product from step (h) (280 mg) was dissolved in dry DMF (10 ml) and treated with 3-bromothiophene-2-carbonitrile (157 mg) followed by sodium hydride (60% in oil, 35 mg) under nitrogen. The reaction mixture was stirred for 24 h, poured into water and extracted with ethyl acetate (3 x 50 ml). The combined organic layers were washed with brine and dried (MgSO₄). The solvent was evaporated and the residue purified by chromatography (silica, 10% ethyl acetate/isohexane as eluent) to give the sub-title compound (250 mg) as an oil. NMR shows the compound to be a mixture of rotomers and the spectrum exhibits very broad peaks.

¹H NMR 300MHz (CDCl₃) 7.20-7.45 (6H, m), 6.60-7.00 (1H, m), 4.10-4.50 (2H, m), 3.70-3.95 (1H, m), 3.40-3.70 (1H, m), 2.25-35 (1H, m), 2.05-2.20 (1H, m), 1.40-1.69 (15H, m).

### k)3-[[(1R,3S)-3-Amino-4-hydroxy-1-phenylbutyllthiol-2-thiophenecarbonitrile oxalate

To a solution of the product from step (j) (240 mg) in methanol (10 ml) was added 4M HCl in dioxane (10 ml). The mixture was stirred at 20 °C for 2 h and the solvent was removed *in vacuo.* The residue was partitioned between aqueous sodium bicarbonate solution and ethyl acetate. The aqueous phase was extracted with ethyl acetate (2 x 50 ml) and the combined extracts were washed with brine, dried (MgSO₄), filtered and concentrated in *vacuo.* The residue was dissolved in ethyl acetate and a solution of oxalic acid in diethyl ether added. The solution was filtered and dried to give the title compound (140 mg) as a white solid.

MS (APCI+ve) ^{m}/z 305 [M(+H)]⁺.
¹H NMR 400MHz (DMSO-*d₆*) 8.04 (1H, d), 7.24-7.34 (6H, m), 4.82 (1H, t), 3.52 (1H, m), 3.42 (1H, m), 2.99 (1H, m), 2.25 (1H, m), 2.10 (1H, m).

### Example 2

### 3-[[(1R,3S)-3-Amino-4-hydroxy-1-phenylbutyllthiol-5-methyl-2-thio-phenecarbonitrile oxalate

### a) 3-Bromo-5-methyl-2-thiophenecarbonitrile

To a suspension of 3-bromo-5-methyl-2-thiophenecarboxaldehyde (500 mg) in water (10 ml) was added hydroxylamine-O-sulfonic acid (330 mg). The mixture was heated to 50 °C for 12 h and allowed to cool. The mixture was then extracted with ethyl acetate (3 x 50 ml). The combined organic layers were washed (brine), dried (MgSO₄), filtered and evaporated to leave the sub-title compound (410 mg) as a brown solid.

¹H NMR 300MHz (CDCl₃) 6.79 (1H, s), 2.54 (3H, s).

### b) 1,1-Dimethylethyl (4S)-4-[(2R)-2-[(2-cyano-5-methyl-3-thienyl)thio]-2-phenylethyl]-2,2-dimethyl-3-oxazolidinecarboxylate

The product from step (a) (170 mg) was dissolved in dry DMF (10 ml) and treated with the product from Example 1 step (h) (280 mg) followed by caesium carbonate (270 mg) under nitrogen. The reaction mixture was stirred for 24 h, poured into water and extracted with ethyl acetate (3 x 50 ml). The combined organic layers were washed with brine and dried (MgSO₄). The solvent was evaporated and the residue purified by chromatography (silica, 10% ethyl acetate/isohexane as eluent) to give the sub-title compound (100 mg) as an oil.

MS (APCI+ve) ^{m}/z 359 [M+H-BOC]⁺.

### c) 3-[[(1R,3S)-3-Amino-4-hydroxy-1-phenylbutyl]thio]-5-methyl-2-thiophenecarbonitrile oxalate

The title compound (60 mg) was prepared by the method of Example 1 step (k) using the product from step (b) above (100 mg).

MS (APCI+ve) ^{m}/z 319 [M(+H)]⁺.
¹H 400MHz (DMSO-*d₆*) 7.32 (5H, m), 7.03 (1H, s), 4.80 (1H, m), 3.51 (1H, m), 3.41 (1H, m), 2.96 (1H, m), 2.50 (3H, s), 2.25 (1H, m), 2.11 (1H, m).

### Screens

The pharmacological activity of compounds according to the invention was tested in the following screens.

### Screen 1

The activity of compounds of formula (I), or a pharmaceutically acceptable salt, enantiomer or racemate thereof, may be screened for nitric oxide synthase inhibiting activity by a procedure based on that of Förstermann et al., Eur. J. Pharm., 1992, 225, 161-165. Nitric oxide synthase converts ³H-L-arginine into ³H-L-citrulline which can be separated by cation exchange chromatography and quantified by liquid scintillation counting.

Enzyme is prepared, after induction, from the cultured murine macrophage cell line J774A-1 (obtained from the laboratories of the Imperial Cancer Research Fund). J774A-1 cells are cultured in Dulbeccos Modified Eagles Medium (DMEM) supplemented with 10% foetal bovine serum, 4 mM L-glutamine and antibiotics (100 units/ml penicillin G, 100 mg/ml streptomycin & 0.25 mg/ml amphotericin B). Cells are routinely grown in 225 cm³ flasks containing 35 ml medium kept at 37 °C and in a humidified atmosphere containing 5% CO₂.

Nitric oxide synthase is produced by cells in response to interferon-g (IFNg) and lipopolysaccharide (LPS). The medium from confluent culture flasks is removed and replaced with 25 ml (per flask) of fresh medium containing 1 mg/ml LPS and 10 units/ml IFNg. After a period of 17-20 hours in culture, harvesting of cells is accomplished by scraping the cell sheet from the flask surface into the culture medium. Cells are collected by centrifugation (1000 g for 10 minutes) and lysate prepared by adding to the cell pellet a solution containing 50 mM Tris-HCI (pH 7.5 at 20 °C), 10% (v/v) glycerol, 0.1% (v/v) Triton-X-100, 0.1 mM dithiothreitol and a cocktail of protease inhibitors comprising leupeptin (2 mg/ml), soya bean trypsin inhibitor (10 mg/ml), aprotinin (5 mg/ml) and phenylmethylsulphonyl fluoride (50 mg/ml).

For the assay, 25 µl of substrate cocktail (50 mM Tris-HCI (pH 7.5 at 20 °C), 400 µM NADPH, 20 µM flavin adenine dinucleotide, 20 µM flavin mononucleotide, 4 µM tetrahydrobiopterin, 12 µM L-arginine and 0.025 mCi L-[³H] arginine) is added to wells of a 96 well filter plate (0.45µM pore size) containing 25 µl of a solution of test compound in 50 mM Tris-HCI. The reaction is started by adding 50 µl of cell lysate (prepared as above) and after incubation for 1 hour at room temperature is terminated by addition of 50 µl of an aqueous solution of 3 mM nitroarginine and 21 mM EDTA.

Labelled L-citrulline is separated from labelled L-arginine using Dowex AG-50W. 150 µl of a 25% aqueous slurry of Dowex 50W (Na⁺ form) is added to the assay after which the whole is filtered into 96 well plates. 75 µl of filtrate is sampled and added to wells of 96 well plates containing solid scintillant. After allowing the samples to dry the L-citrulline is quantified by scintillation counting.

In a typical experiment basal activity is 300 dpm per 75 µl sample which is increased to 1900 dpm in the reagent controls. Compound activity is expressed as IC₅₀ (the concentration of drug substance which gives 50% enzyme inhibition in the assay) and aminoguanidine, which gives an IC₅₀ (50% inhibitory concentration) of 10 µM, is tested as a standard to verify the procedure. Compounds are tested at a range of concentrations and from the inhibitions obtained IC₅₀ values are calculated. Compounds that inhibit the enzyme by at least 25% at 100 µM are classed as being active and are subjected to at least one retest.

In the above screen, the compounds of Examples 1 to 2 were tested and gave IC₅₀ values of less than 10 µM indicating that they are expected to show useful therapeutic activity.

### Screen 2

Recombinant human NO synthases (iNOS, eNOS & nNOS) were expressed in *E. coli* and lysates were prepared in Hepes buffer (pH 7.4) containing co-factors (FAD, FMN, H₄B), protease inhibitors, lysozyme and the detergent, CHAPS. These preparations were used, at suitable dilution, to assess inhibition of the various isoforms. Inhibition of NOS was determined by measuring the formation of L-[³H]citrulline from L-[³H]arginine using an adaptation of the method of Förstermann *et al.*⁹ Enzyme assays were performed in the presence of 3 µM [³H]arginine, 1 mM NADPH and other co-factors required to support NOS activity (FAD, FMN, H₄B, calmodulin, Ca²⁺). Since various NOS inhibitors have been reported to exhibit slow binding kinetics, or to inactivate the enzyme in a time dependent manner, enzyme and inhibitor were pre-incubated for 60 min in the presence of NADPH before addition of arginine to initiate the reaction. Incubations continued for a further 60 min before the assays were quenched and [³H]citrulline separated from unreacted substrate by chromatography on Dowex-50W resin in a 96-well format.

In the above screen, the compounds of Examples 1 to 2 were tested and gave IC₅₀ values of less than 10 µM against the iNOS enzyme indicating that they are expected to show useful therapeutic activity.

### Screen 3

Compounds also show activity against the human form of induced nitric oxide synthase as can be demonstrated in the following assay.

The human colorectal carcinoma cell line, DLD-1 (obtained from the European Collection of Animal Cell Culture - cell line number 90102540) was routinely grown in RPMI 1640 supplemented with 10%(v/v) foetal bovine serum, and 2mM L-glutamine, at 37 °C in 5% CO₂.

Nitric oxide synthase was induced in cells by addition of medium containing human recombinant gamma-IFN (1000 units/ml), TNF-alpha (200 U/ml), IL-6 (200 U/ml) and IL-1-beta (250 U/ml). After incubation for 18 hours at 37°C, the medium was removed and the cells washed with warm phosphate buffered saline. Cells were incubated for a further 5 hours at 37 °C / 5% CO₂ in RPMI 1640 containing 100µM L-arginine and 100µM verapamil-HCl in the presence and absence of test compounds.

Nitrite accumulation was determined by mixing an equal volume of culture media with Griess reagent (10 mg/ml sulphanilamide, 1 mg *N*-(1-naphthyl)ethylenediamine in 1 ml 2.5% (v/v) phosphoric acid). Inhibition in the presence of compounds was calculated relative to the nitrite levels produced by untreated cells. IC₅₀ values were estimated from a semi-log plot of % inhibition versus concentration of compound.

In this screen the compounds of Examples 1 to 2 gave IC₅₀ values of less than 100 µM, indicating that they are predicted to show useful therapeutic activity.

## Claims

1. A compound of formula (I) 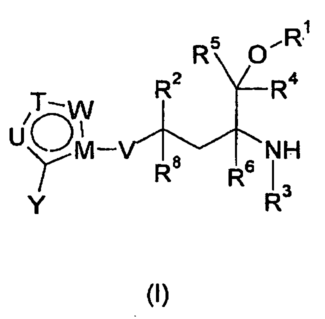 wherein:
Y represents C1 to 4 alkyl, C1 to 4 alkoxy, halogen, CN, C≡CH, NO₂, CH₂OH, CHO, COCH₃, NH₂, NHCHO, NHCOCH3 or NHSO₂CH₃; said alkyl or alkoxy group being optionally further substituted by one or more fluorine atoms;
T, U and W independently represent CX, N, NR¹³, O or S(O)ₘ, except that at least one of T, U and W must represent a heteroatom and except that not more than one of T, U and W may represent NR¹³, O or S(O)ₘ; m represents an integer 0, 1 or 2; and each X group independently represents H, C 1 to 4 alkyl, C 1 to 4 alkoxy, halogen, OH, SH, CN, C≡CH, N(R¹⁴)₂, NO₂, CH₂OH, CHO, COCH₃ or NHCHO; said alkyl or alkoxy group being optionally further substituted by one or more fluorine atoms;
V represents O or S(O)ₙ;
n represents an integer 0, 1 or 2;
M represents C;
R¹ and R⁸ independently represent H or Me.
R² represents C1 to 4 alkyl, C2 to 4 alkenyl, C2 to 4 alkynyl, C3 to 6 cycloalkyl or a 4 to 8 membered saturated heterocyclic ring incorporating one heteroatom selected from O, S and N; any of said groups being optionally further substituted by C 1 to 4 alkyl, C 1 to 4 alkoxy, C1 to 4 alkylthio, C3 to 6 cycloalkyl, halogen or phenyl; said phenyl group being optionally further substituted by one or more substituents selected independently from halogen, C1 to 4 alkyl, C 1 to 4 alkoxy, CF₃, OCF₃, CN or NO₂;
or R² represents phenyl or a five or six membered aromatic heterocyclic ring containing 1 to 3 heteroatoms independently selected from O, S and N; said phenyl or aromatic heterocyclic ring being optionally substituted by one or more substituents selected independently from halogen, C1 to 4 alkyl, C1 to 4 alkoxy, OH, CN, NO₂ or NR⁹R¹⁰; said alkyl or alkoxy group being optionally further substituted by one or more fluorine atoms;
R³ represents H, C1 to 4 alkyl or C3 to 6 cycloalkyl; said alkyl group being optionally substituted by C1 to 4 alkoxy, halogen, hydroxy, NR¹¹R¹², phenyl or a five or six membered aromatic or saturated heterocyclic ring containing 1 to 3 heteroatoms independently selected from O, S and N; said phenyl or aromatic heterocyclic ring being optionally further substituted by halogen, C1 to 4 alkyl, C1 to 4 alkoxy, CF₃, OCF₃, CN or NO₂;
R⁷ and R¹⁴ independently represent H or C1 to 2 alkyl;
R⁴, R⁵, R⁶, R⁹, R¹⁰, R¹¹ and R¹² independently represent H or C1 to 4 alkyl;
R¹³ represents H, C1 to 4 alkyl, CHO, COCH₃, SO₂CH₃ or CF₃;
or a pharmaceutically acceptable salt thereof.

2. A compound of formula (I), according to Claim 1, wherein V represents S(O)ₙ and n represents 0.

3. A compound according to Claim 1 or 2 wherein Y represents CN.

4. A compound of formula (I), according to Claim 1, which is:
3-[[(1*R*,3*S*)-3-amino-4-hydroxy-1-phenylbutyl]thio]-2-thiophenecarbonitrile;
3-[[(1R,3S)-3-amino-4-hydroxy-1-phenylbutyl]thio]-5-methyl-2-thiophenecarbonitrile;
or a pharmaceutically acceptable salt, enantiomer or racemate thereof.

5. A compound of formula (I), according to any one of Claims 1 to 4, or a pharmaceutically acceptable salt thereof, for use as a medicament.

6. A pharmaceutical composition comprising a compound of formula (I) according to any one of Claims 1 to 4, or a pharmaceutically acceptable salt thereof, in admixture with a pharmaceutically acceptable adjuvant, diluent or carrier.

7. The use of a compound of formula (I) according to any one of Claims 1 to 4, or a pharmaceutically acceptable salt thereof, in the manufacture of a medicament for the treatment or prophylaxis of human diseases or conditions in which inhibition of nitric oxide synthase activity is beneficial.

8. The use as claimed in Claim 7 wherein it is predominantly inducible nitric oxide synthase that is inhibited.

9. The use of a compound of formula (I) as defined in any one of Claims 1 to 4, or a pharmaceutically acceptable salt thereof, in the manufacture of a medicament, for the treatment or prophylaxis of inflammatory diseases.

10. The use as claimed in Claim 9 wherein the disease is inflammatory bowel disease.

11. The use as claimed in Claim 9 wherein the disease is rheumatoid arthritis.

12. The use as claimed in Claim 9 wherein the disease is osteoarthritis.

13. The use of a compound of formula (I) as defined in any one of Claims 1 to 4, or a pharmaceutically acceptable salt thereof, in the manufacture of a medicament, for the treatment or prophylaxis of pain.

14. The use of a compound of formula (I) as defined in any one of Claims 1 to 4, or a pharmaceutically acceptable salt thereof, in combination with a COX-2 inhibitor, in the manufacture of a medicament, for the treatment or prophylaxis of inflammatory diseases.

15. A process for the preparation of a compound of formula (I), as defined in any one of Claims 1 to 4, or a pharmaceutically acceptable salt, enantiomer or racemate thereof, wherein the process comprises:
(a) reaction of a compound of formula (II) 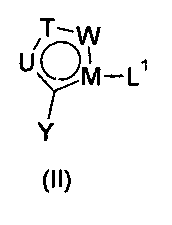 wherein T, U, W, Y and M are as defined in Claim 1 and L¹ represents a leaving group, with a compound of formula (III) 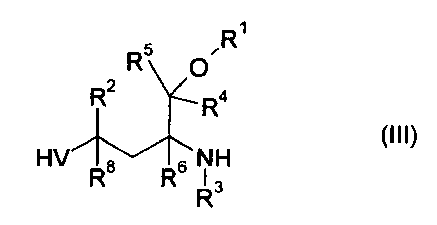 wherein R¹, R², R³, R⁴, R⁵, R⁶, R⁸ and V are as defined in Claim 1; or
(b) reaction of a compound of formula (IV) 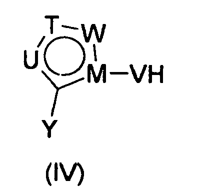
wherein T, U, W, M, Y and V are as defined in Claim 1,
with a compound of formula (V) 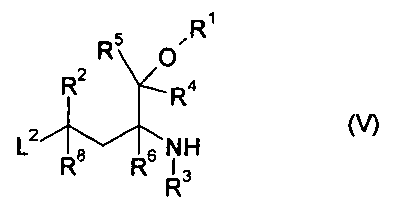 wherein R¹, R², R³, R⁴, R⁵, R⁶ and R⁸ are as defined in Claim 1 and L² is a leaving group;
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

## Patentansprüche

1. Verbindung der Formel (I) 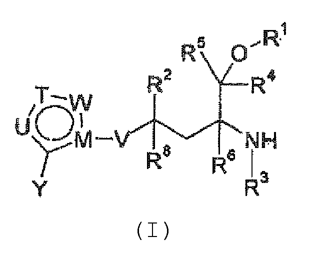 worin:
Y für C₁₋₄-Alkyl, C₁₋₄-Alkoxy, Halogen, CN, C=CH, NO₂, CH₂OH, CHO, COCH₃, NH₂, NHCHO, NHCOCH₃ oder NHSO₂CH₃ steht; wobei die Alkyl- oder Alkoxygruppe gegebenenfalls ferner durch ein oder mehrere Fluoratome substituiert ist;
T, U und W unabhängig voneinander für CX, N, NR¹³, O oder S(O)ₘ stehen, außerd daß mindestens eine der Variablen T, U und W für ein Heteroatom stehen muß und höchstens eine der Variablen T, U und W für NR¹³, 0 oder S(O)ₘ stehen darf; m für eine ganze Zahl mit einem Wert von 0, 1 oder 2 steht und jede Gruppe X unabhängig voneinander für H, C₁₋₄-Alkyl, C₁₋₄-Alkoxy, Halogen, OH, SH, CN, C≡CH, N(R¹⁴)₂, NO₂, CH₂OH, CHO, COCH₃ oder NHCHO steht; wobei die Alkyl- oder Alkoxygruppe gegebenenfalls ferner durch ein oder mehrere Fluoratome substituiert ist;
V für O oder S(O)ₙ steht;
n für eine ganze Zahl mit einem Wert von 0, 1 oder 2 steht;
M für C steht;
R¹ und R⁸ unabhängig voneinander für H oder Me stehen;
R² für C₁₋₄-Alkyl, C₂₋₄-Alkenyl, C₂₋₄-Alkinyl, C₃₋₆-Cycloalkyl oder einen 4- bis 8-gliedrigen heterocyclischen Ring mit einem unter 0, S und N ausgewählten Heteroatom steht; wobei jede dieser Gruppen gegebenenfalls ferner durch C₁₋₄-Alkyl, C₁₋₄-Alkoxy, C₁₋₄-Alkylthio, C₃₋₆-Cycloalkyl, Halogen oder Phenyl substituiert ist; wobei die Phenylgruppe gegebenenfalls ferner durch einen oder mehrere unabhängig voneinander unter Halogen, C₁₋₄-Alkyl, C₁₋₄-Alkoxy, CF₃, OCF₃, CN oder NO₂ ausgewählte Substituenten substituiert ist;
oder R² für Phenyl oder einen fünf- oder sechsgliedrigen aromatischen heterocyclischen Ring mit 1 bis 3 unabhängig voneinander unter 0, S und N ausgewählten Heteroatomen steht; wobei das Phenyl oder der aromatische heterocyclische Ring gegebenenfalls durch einen oder mehrere unabhängig voneinander unter Halogen, C₁₋₄-Alkyl, C₁₋₄-Alkoxy, OH, CN, NO₂ oder NR⁹R¹⁰ ausgewählte Substituenten substituiert ist; wobei die Alkyl- oder Alkoxygruppe gegebenenfalls ferner durch ein oder mehrere Fluoratome substituiert ist;
R³ für H, C₁₋₄-Alkyl oder C₃₋₆-Cycloalkyl steht; wobei die Alkylgruppe gegebenenfalls durch C₁₋₄-Alkoxy, Halogen, Hydroxy, NR¹¹R¹², Phenyl oder einen fünf- oder sechsgliedrigen aromatischen oder gesättigten heterocyclischen Ring mit 1 bis 3 unabhängig voneinander unter 0, S und N ausgewählten Heteroatomen substituiert ist; wobei das Phenyl oder der aromatische heterocyclische Ring gegebenenfalls ferner durch Halogen, C₁₋₄-Alkyl, C₁₋₄-Alkoxy, CF₃, OCF₃, CN oder NO₂ substituiert ist;
R⁷ und R¹⁴ unabhängig voneinander für H oder C₁₋₂-Alkyl stehen;
R⁴, R⁵, R⁶, R⁹, R¹⁰, R¹¹ und R¹² unabhängig voneinander für H oder C₁₋₄-Alkyl stehen;
R¹³ für H, C₁₋₄-Alkyl, CHO, COCH₃, SO₂CH₃ oder CF₃ steht;
oder ein pharmazeutisch annehmbares Salz davon.

2. Verbindung der Formel (I) nach Anspruch 1, in der V für S(O)ₙ steht und n für 0 steht.

3. Verbindung nach Anspruch 1 oder 2, in der Y für CN steht.

4. Verbindung der Formel (I) nach Anspruch 1, bei der es sich um
3-[[(1*R*,3*S*)-3-Amino-4-hydroxy-1-phenylbutyl]thio]-2-thiophencarbonitril,
3-[[(1*R*,3*S*)-3-Amino-4-hydroxy-1-phenylbutyl]thio]-5-methyl-2-thiophencarbonitril
oder ein pharmazeutisch annehmbares Salz, Enantiomer oder Racemat davon handelt.

5. Verbindung der Formel (I) nach einem der Ansprüche 1 bis 4 oder ein pharmazeutisch annehmbares Salz davon zur Verwendung als Arzneimittel.

6. Pharmazeutische Zusammensetzung, enthaltend eine Verbindung der Formel (I) nach einem der Ansprüche 1 bis 4 oder ein pharmazeutisch annehmbares Salz davon in Abmischung mit einem pharmazeutisch annehmbaren Hilfsstoff, Verdünnungsmittel oder Träger.

7. Verwendung einer Verbindung der Formel (I) nach einem der Ansprüche 1 bis 4 oder eines pharmazeutisch annehmbaren Salzes davon bei der Herstellung eines Arzneimittels zur Behandlung oder Prophylaxe von humanen Erkrankungen oder Leiden, bei denen eine Inhibierung der Stickstoffmonoxidsynthaseaktivität von Nutzen ist.

8. Verwendung nach Anspruch 7, bei der vorwiegend die induzierbare Stickstoffmonoxidsynthase inhibiert wird.

9. Verwendung einer Verbindung der Formel (I) gemäß einem der Ansprüche 1 bis 4 oder eines pharmazeutisch annehmbaren Salzes davon bei der Herstellung eines Arzneimittels zur Behandlung oder Prophylaxe von entzündlichen Erkrankungen.

10. Verwendung nach Anspruch 9, bei der es sich bei der Erkrankung um entzündliche Darmerkrankung handelt.

11. Verwendung nach Anspruch 9, wobei es sich bei der Erkrankung um rheumatoide Arthritis handelt.

12. Verwendung nach Anspruch 9, wobei es sich bei der Erkrankung um Osteoarthritis handelt.

13. Verwendung einer Verbindung der Formel (I) gemäß einem der Ansprüche 1 bis 4 oder eines pharmazeutisch annehmbaren Salzes davon bei der Herstellung eines Arzneimittels zur Behandlung oder Prophylaxe von Schmerzen.

14. Verwendung einer Verbindung der Formel (I) gemäß einem der Ansprüche 1 bis 4 oder eines pharmazeutisch annehmbaren Salzes davon in Kombination mit einem COX-2-Inhibitor bei der Herstellung eines Arzneimittels zur Behandlung oder Prophylaxe von entzündlichen Erkrankungen.

15. Verfahren zur Herstellung einer Verbindung der Formel (I) gemäß einem der Ansprüche 1 bis 4 oder eines pharmazeutisch annehmbaren Salzes, Enantiomers oder Racemats davon, bei dem man:
(a) eine Verbindung der Formel (II) 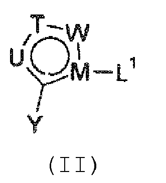 worin T, U, W, Y und M die in Anspruch 1 angegebene Bedeutung besitzen und L¹ für eine Abgangsgruppe steht,
mit einer Verbindung der Formel (III) 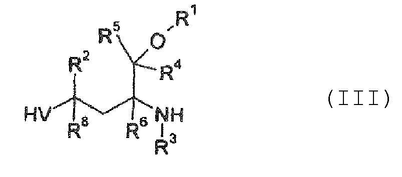 worin R¹, R², R³, R⁴, R⁵, R⁶, R⁸ und V die in Anspruch 1 angegebene Bedeutung besitzen, umsetzt; oder
(b) eine Verbindung der Formel (IV) 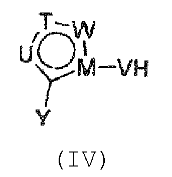
worin T, U, W, M, Y und V die in Anspruch 1 angegebene Bedeutung besitzen, mit einer Verbindung der Formel (V) 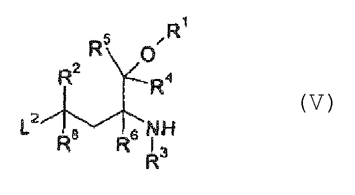 worin R¹, R², R³, R⁴, R⁵, R⁶ und R⁸ die in Anspruch 1 angegebene Bedeutung besitzen und L² für eine Abgangsgruppe steht, umsetzt;
und, falls gewünscht oder erforderlich, die erhaltene Verbindung der Formel (I) oder ein anderes Salz davon in ein pharmazeutisch annehmbares Salz davon umwandelt; oder eine Verbindung der Formel (I) in eine andere Verbindung der Formel (I) umwandelt; und, falls gewünscht, die erhaltene Verbindung der Formel (I) in ein optisches Isomer davon umwandelt.

## Revendications

1. Composé de formule (I) 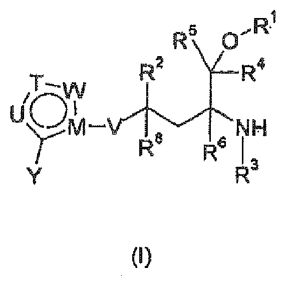 dans laquelle:
Y représente alkyle en C1 à 4, alcoxy en C1 à 4, halogène, CN, C=CH, NO₂, CH₂OH, CHO, COCH₃, NH₂, NHCHO, NHCOCH₃ ou NHSO₂CH₃ ; ledit groupement alkyle ou alcoxy étant éventuellement en outre substitué par un ou plusieurs atomes de fluor ;
T, U et W représentent indépendamment CX, N, NR¹³, O ou S(O)ₘ, sauf qu'au moins l'un de T, U et W doit représenter un hétéroatome et sauf qu'au plus un de T, U et W peut représenter NR¹³, 0 ou S(O)ₘ; m représente un entier valant 0, 1 ou 2 ; et chaque groupement X représente indépendamment H, alkyle en C1 à 4, alcoxy en C1 à 4, halogène, OH, SH, CN, C=CH, N(R¹⁴)₂, NO₂, CH₂OH, CHO, COCH₃ ou NHCHO ; ledit groupement alkyle ou alcoxy étant éventuellement en outre substitué par un ou plusieurs atomes de fluor ;
V représente O ou S(O)ₙ ;
n représente un entier valant 0, 1 ou 2 ;
M représente C ;
R¹ et R⁸ représentent indépendamment H ou Me ;
R² représente alkyle en C1 à 4, alcényle en C2 à 4, alcynyle en C2 à 4, cycloalkyle en C3 à 6, ou un cycle hétérocyclique saturé de 4 à 8 chaînons incorporant un hétéroatome choisi parmi O, S et N ; l'un quelconque desdits groupements étant éventuellement en outre substitué par alkyle en C1 à 4, alcoxy en C1 à 4, alkylthio en C1 à 4, cycloalkyle en C3 à 6, halogène ou phényle; ledit groupement phényle étant éventuellement en outre substitué par un ou plusieurs substituants choisis indépendamment parmi halogène, alkyle en C1 à 4, alcoxy en C1 à 4, CF₃, OCF₃, CN ou NO₂;
ou R² représente phényle ou un cycle hétérocyclique aromatique à cinq ou six chaînons contenant 1 à 3 hétéroatomes choisis indépendamment parmi O, S et N ; ledit phényle ou cycle hétérocyclique aromatique étant éventuellement substitué par un ou plusieurs substituants choisis indépendamment parmi halogène, alkyle en C1 à 4, alcoxy en C1 à 4, OH, CN, NO₂ ou NR⁹R¹⁰ ; ledit groupement alkyle ou alcoxy étant éventuellement en outre substitué par un ou plusieurs atomes de fluor ;
R³ représente H, alkyle en C1 à 4 ou cycloalkyle en C3 à 6 ; ledit groupement alkyle étant éventuellement substitué par alcoxy en C1 à 4, halogène, hydroxy, NR¹¹R¹², phényle ou un cycle hétérocyclique aromatique ou saturé à cinq ou six chaînons contenant 1 à 3 hétéroatomes choisis indépendamment parmi 0, S et N ; ledit phényle ou cycle hétérocyclique aromatique étant éventuellement en outre substitué par halogène, alkyle en C1 à 4, alcoxy en C1 à 4, CF₃, OCF₃, CN ou NO₂ ;
R⁷ et R¹⁴ représentent indépendamment H ou alkyle en C1 à 2 ;
R⁴, R⁵, R⁶, R⁹, R¹⁰, R¹¹ et R¹² représentent indépendamment H ou alkyle en C1 à 4 ;
R¹³ représente H, alkyle en C1 à 4, CHO, COCH₃, SO₂CH₃ ou CF₃ ;
ou un sel pharmaceutiquement acceptable de celui-ci.

2. Composé de formule (I), selon la revendication 1, **caractérisé en ce que** V représente S(O)ₙ et n représente 0.

3. Composé selon la revendication 1 ou 2, **caractérisé en ce que** Y représente CN.

4. Composé de formule (I), selon la revendication 1, **caractérisé en ce qu'**il s'agit :
du 3-[[(1*R*,3*S*)-3-amino-4-hydroxy-1-phénylbutyl]-thio]-2-thiophènecarbonitrile ;
du 3-[[(1*R*,3*S*)-3-amino-4-hydroxy-1-phénylbutyl]-thio]-5-méthyl-2-thiophènecarbonitrile ;
ou d'un sel, d'un énantiomère ou d'un racémate pharmaceutiquement acceptable de celui-ci.

5. Composé de formule (I), selon l'une quelconque des revendications 1 à 4, ou un sel pharmaceutiquement acceptable de celui-ci, destiné à être utilisé comme médicament.

6. Composition pharmaceutique comprenant un composé de formule (I) selon l'une quelconque des revendications 1 à 4, ou un sel pharmaceutiquement acceptable de celui-ci, en mélange avec un adjuvant, un diluant ou un support pharmaceutiquement acceptable.

7. Utilisation d'un composé de formule (I) selon l'une quelconque des revendications 1 à 4, ou d'un sel pharmaceutiquement acceptable de celui-ci, dans la fabrication d'un médicament destiné au traitement ou à la prophylaxie de maladies ou d'affections humaines dans lesquelles l'inhibition de l'activité de l'oxyde nitrique synthétase est bénéfique.

8. Utilisation selon la revendication 7, **caractérisée en ce que** c'est principalement l'oxyde nitrique synthétase inductible qui est inhibé.

9. Utilisation d'un composé de formule (I), tel que défini dans l'une quelconque des revendications 1 à 4, ou d'un sel pharmaceutiquement acceptable de celui-ci, dans la fabrication d'un médicament destiné au traitement ou à la prophylaxie de maladies inflammatoires.

10. Utilisation selon la revendication 9, **caractérisée en ce que** la maladie est l'affection abdominale inflammatoire.

11. Utilisation selon la revendication 9, **caractérisée en ce que** la maladie est la polyarthrite rhumatoïde.

12. Utilisation selon la revendication 9, **caractérisée en ce que** la maladie est l'arthrose.

13. Utilisation d'un composé de formule (I), tel que défini dans l'une quelconque des revendications 1 à 4, ou d'un sel pharmaceutiquement acceptable de celui-ci, dans la fabrication d'un médicament destiné au traitement ou à la prophylaxie de la douleur.

14. Utilisation d'un composé de formule (I), tel que défini dans l'une quelconque des revendications 1 à 4, ou d'un sel pharmaceutiquement acceptable de celui-ci, en combinaison avec un inhibiteur de COX-2, dans la fabrication d'un médicament destiné au traitement ou à la prophylaxie de maladies inflammatoires.

15. Procédé de préparation d'un composé de formule (I), tel que défini dans l'une quelconque des revendications 1 à 4, ou d'un sel, d'un énantiomère ou d'un racémate pharmaceutiquement acceptable de celui-ci,
**caractérisé en ce qu'**il comprend :
(a) la réaction d'un composé de formule (II) : 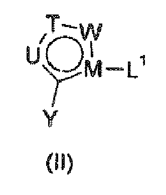 dans laquelle T, U, W, Y et M sont tels que définis dans la revendication 1 et L¹ représente un groupe partant,
avec un composé de formule (III) : 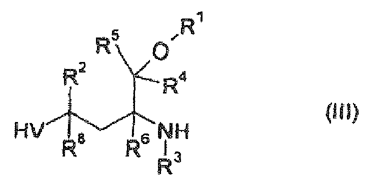 dans laquelle R¹, R², R³, R⁴, R⁵, R⁶, R⁸ et V sont tels que définis dans la revendication 1 ; ou
(b) la réaction d'un composé de formule (IV) : 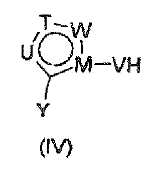
dans laquelle T, U, W, M, Y et V sont tels que définis dans la revendication 1,
avec un composé de formule (V) : 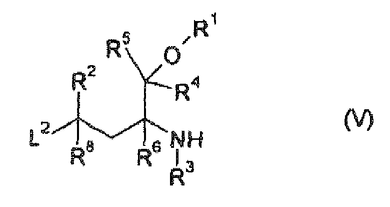 dans laquelle R¹, R², R³, R⁴, R⁵, R⁶ et R⁸ sont tels que définis dans la revendication 1 et L² est un groupe partant ;
et, si on le souhaite ou s'il est nécessaire, la transformation du composé résultant de formule (I), ou d'un autre sel de celui-ci, en un sel pharmaceutiquement acceptable de celui-ci ; ou la transformation d'un composé de formule (I) en un autre composé de formule (I) ; et, si on le souhaite, la transformation du composé résultant de formule (I) en un isomère optique de celui-ci.
